# FASCICULE DE BREVET EUROPEEN

(11) **EP 2 227 179 B1**
(45) Date de publication et mention de la délivrance du brevet: **27.04.2016**
(21) Numéro de dépôt: 08871270.8
(22) Date de dépôt: 05.11.2008
(51) Int. Cl.: A61F 2/34, A61F 2/30, A61F 2/46, A61B 17/80

(54) **ARMATURE DE FOND DE COTYLE POUR PROTHÈSE DE HANCHE**
VERSTÄRKUNG FÜR DIE BASIS EINER HÜFTGELENKPFANNE IN EINER HÜFTPROTHESE
REINFORCEMENT FOR THE BASE OF AN ACETABULAR CUP FOR A HIP PROSTHESIS

(30) Priorité: 05.11.2007 US 996170 P
(43) Date de publication de la demande: 15.09.2010
(73) Titulaire: Vielpeau, Claude, 14200 Hérouville St Clair (FR); Hulet, Christophe, 14000 Caen (FR); Girardin, Philippe, 42600 Lezigneux (FR); Bonnard, Olivier, 69330 Meyzieu (FR); Noyer, Daniel, 38200 Luzinay (FR); Cypres, Alain, 42190 St Nizier sous Charlieu (FR); Fiquet, Arnaud, 69300 Caluire (FR); Bauchu, Philippe, 69002 Lyon (FR); Roy, Christophe, 26300 Chatuzange Le Goubet (FR); Moulin, Jean-Pierre, 69006 Lyon (FR)
(72) Inventeur: Vielpeau, Claude, 14200 Hérouville St Clair (FR); Hulet, Christophe, 14000 Caen (FR); Girardin, Philippe, 42600 Lezigneux (FR); Bonnard, Olivier, 69330 Meyzieu (FR); Noyer, Daniel, 38200 Luzinay (FR); Cypres, Alain, 42190 St Nizier sous Charlieu (FR); Fiquet, Arnaud, 69300 Caluire (FR); Bauchu, Philippe, 69002 Lyon (FR); Roy, Christophe, 26300 Chatuzange Le Goubet (FR); Moulin, Jean-Pierre, 69006 Lyon (FR)
(74) Mandataire: Verriest, Philippe
(86) Numéro de dépôt international: PCT/FR2008/001554
(87) Numéro de publication internationale: WO 2009/092910

(56) Documents cités:
- EP-A- 1 312 322
- DE-A1- 19 841 251
- FR-A- 2 827 504
- FR-A- 2 842 096
- FR-A- 2 854 057
- US-A- 5 037 424
- US-A- 5 171 313
- US-A- 5 584 837

## Description

La présente invention concerne une armature pour fond de cotyle de prothèse de hanche, un insert utilisable pour séparer une armature de fond de cotyle d'une prothèse de hanche ainsi qu'un instrument chirurgical pour la fixation d'une armature de fond de cotyle au sein d'une cavité acétabulaire.

La mise en place d'une prothèse de hanche s'avère difficile lorsque l'os du bassin, et en particulier la partie de l'os délimitant la cavité acétabulaire, présente une dégradation osseuse menant à un défect osseux. En effet, une telle dégradation diminue les possibilités d'appui de l'implant cotyloïdien, tel qu'une prothèse de hanche, sur l'os du bassin. Dans un tel cas, une armature de fond de cotyle, destinée d'une part à être fixée à l'os du bassin au niveau de la cavité acétabulaire et d'autre part à recevoir l'implant cotyloïdien ou prothèse de hanche, est nécessaire. Une telle armature doit permettre un scellement en position et avec une bonne stabilité de l'implant cotyloïdien malgré un défect osseux, même significatif, du bassin.

Des dispositifs de telles armatures de fond de cotyle existent en différentes configurations, notamment sous la forme d'une coquille hémisphérique percée de trous, ou une croix galbée sensiblement à la forme de la cavité acétabulaire destinées à être cimentées entre la cavité osseuse et la cupule d'articulation de la prothèse de hanche.

En particulier, les armatures de fond de cotyle en forme de croix galbée présentent généralement quatre pattes concentriques (les branches de la croix), réparties de façon régulière ou non autour de l'axe de révolution correspondant à l'axe de la demi-sphère formée par la cavité acétabulaire : parmi ces quatre pattes, deux d'entre elles, opposées l'une à l'autre, ie séparées par un angle de 180°, présentent à leur extrémité une partie en forme de crochet : l'un de ces crochets est destiné à être engagé dans le trou obturateur du bassin tandis que l'autre est destiné à être fixé, par exemple à l'aide de vis dans la partie de l'os délimitant la partie supérieure de la cavité acétabulaire : ces deux pattes assurent ainsi la fixation de l'armature à l'os dans le sens vertical. Les deux autres pattes, également opposées l'une à l'autre, sont destinées à prendre appui sur l'os du bassin et assurent la stabilisation latérale de l'armature. Toutefois, dans le cas ou le défect osseux est important, il peut être nécessaire d'ajuster la longueur de chacune de ces deux pattes à la configuration spécifique de l'os défectueux.

Par ailleurs, du fait de la configuration de l'os du bassin, qui est symétrique par rapport au plan sagittal, la cavité acétabulaire gauche présente une orientation opposée à celle de la cavité acétabulaire droite. Ainsi, une armature de fond de cotyle destinée à être mise en place dans la cavité acétabulaire gauche ne pourra pas être utilisée pour la cavité acétabulaire droite.

La demande de brevet français publiée sous le numéro FR 2 842 096 décrit un moyen de séparation entre une armature pour fond de cotyle et une prothèse acétabulaire. Dans l'art actuel, les armatures en croix pour fond de cotyle présentent soit des pattes de longueur égale, soit des pattes asymétriques nécessitant une armature droite et une armature gauche, respectivement pour la cavité acétabulaire droite et pour la cavité acétabulaire gauche.

Par ailleurs, pour éviter toute métalose ou réaction par contact et friction entre l'armature de fond de cotyle et l'implant cotyloïdien que cette dernière est destinée à recevoir, il est important de prévoir un dispositif permettant la séparation de l'armature et de l'implant cotyloïdien ou prothèse acétabulaire. Le dispositif permettant la séparation de l'armature et d'une prothèse acétabulaire décrit dans FR 2 842 096 est constitué d'une croix de forme identique à celle de l'armature. Un tel dispositif est de fabrication complexe et présente un encombrement important. Il constitue également un apport significatif de corps étranger dans le corps du patient.

Dans les armatures de l'art antérieur, les pattes comportent généralement des fentes : ces fentes peuvent aider à ajuster les pattes sur l'os du bassin mais elles tendent à fragiliser lesdites pattes.

La présente invention est destinée à améliorer les dispositifs d'armature de fond de cotyle de l'art antérieur de la façon décrite ci-après et est définie par les caractéristiques de la revendication 1.

Un premier aspect de l'invention est une armature pour fond de cotyle comprenant une croix galbée comportant au moins quatre pattes concentriques réparties autour de l'axe de révolution correspondant à l'axe de la demi sphère de la cavité acétabulaire, caractérisée en ce qu'au moins une des pattes est autocassable.

Par autocassable, on entend, au sens de la présente demande, une patte pouvant être cassée de façon aisée par un chirurgien sur le site opératoire, préalablement à l'implantation, éventuellement à l'aide d'un outil complémentaire, de façon à détacher de ladite patte une partie de l'extrémité libre de ladite patte. Le but de la présence d'une telle patte autocassable est par la rupture d'une extrémité d'une des pattes de récréer l'asymétrie de la cavité acétabulaire et donc d'éviter d'avoir à disposition un surcroît d'armatures de tailles différentes. Cet auto-cassage se fait préalablement à l'implantation en fonction du côté sélectionné. Ainsi, il n'est pas nécessaire de disposer d'armatures différentes pour la cavité acétabulaire gauche et pour la cavité acétabulaire droite, et ce quelque soit l'état de dégradation de l'os du bassin. Le chirurgien prépare avant l'implantation, à partir d'une armature selon l'invention, en cassant ou pas une ou plusieurs pattes autocassables, l'armature adaptée à la fois à la cavité acétabulaire, gauche ou droite, et à l'état de dégradation de l'os traité, pour la meilleure stabilisation latérale de l'armature.

Selon l'invention, parmi lesdites quatre pattes, au moins une première patte comporte à son extrémité libre une pointe recourbée destinée à être logée dans le trou obturateur du bassin lorsque l'armature est implantée, au moins une deuxième patte, destinée à être positionnée dans la face antéro-externe de la cavité acétabulaire lorsque l'armature est implantée, comporte à son extrémité libre une plaque sensiblement ovalaire et galbée, destinée à être fixée au support osseux, au moins une des troisième et quatrième pattes étant autocassable. Dans une forme de réalisation de l'invention, ladite première patte et ladite deuxième patte sont opposées l'une à l'autre et lesdites troisième et quatrième pattes sont autocassables. Ainsi, par rupture de l'extrémité d'une des troisième et quatrième pattes, le chirurgien recrée l'asymétrie de la cavité acétabulaire. Il peut ainsi, à partir d'une armature selon l'invention, préparer une armature pour cavité acétabulaire gauche ou une armature pour cavité acétabulaire droite, en fonction du cas qu'il est en train de traiter. La nature autocassable des troisième et quatrième pattes permet également au chirurgien d'adapter la structure de l'armature au degré de dégradation de l'os du bassin.

Dans une forme de réalisation de l'invention, chacune de la ou desdites patte(s) autocassable(s) comporte dans la région de son extrémité libre une partie rétrécie autorisant à son endroit la rupture de ladite patte. Ainsi, le chirurgien peut aisément casser ladite patte au niveau de la partie rétrécie de cette dernière. L'extrémité libre de la patte rattachée au reste de la patte par la partie rétrécie est ainsi détachée de la patte et la longueur de la patte est alors adaptée à la l'obliquité (gauche ou droite) de la cavité acétabulaire traitée et au degré de dégradation de l'os du bassin. Pour casser l'extrémité de la patte autocassable, le chirurgien peut se munir d'un outil, tel qu'une simple pince, pour saisir l'extrémité de la patte et exercer un mouvement de levier au niveau de la partie rétrécie pour causer la rupture de la patte à l'endroit de cette partie rétrécie.

Dans une forme de réalisation de l'invention, le dispositif comporte une patte destinée à s'adapter au plan osseux. Pour ce faire, la patte est munie d'une fente permettant de la modeler au support osseux. La fente précitée est caractérisée par un ou plusieurs orifices permettant de dissiper les contraintes lors du modelage de la patte et d'éviter sa rupture. Ces orifices permettent une seconde fonction, à savoir la possibilité de stabiliser provisoirement en per-opératoire l'armature à l'aide de vis temporaires.

Ainsi, selon l'invention, ladite deuxième patte est munie d'une fente longitudinale se terminant, au niveau de ladite plaque ovalaire et galbée, par au moins un orifice de forme sensiblement circulaire. La présence de l'orifice de forme sensiblement circulaire terminant ladite fente au niveau de la plaque ovalaire et galbée permet de dissiper les contraintes lorsque la deuxième patte est modelée pour s'adapter au support osseux : en effet, au cours de cette opération, la deuxième patte est tordue, par exemple à l'aide d'un outil apte à saisir la plaque ovalaire et galbée, pour que ladite plaque ovalaire et galbée puisse prendre appui et être plaquée et être fixée sur le support osseux. Grâce à la présence de l'orifice de forme sensiblement circulaire terminant la fente comme indiqué ci-dessus, la patte ne se rompt pas lorsqu'elle est tordue pour l'opération de fixation de la plaque sur le support osseux.

Dans une forme de réalisation, ladite première patte comporte dans la région de son extrémité libre un trou de forme sensiblement circulaire. Comme il ressortira de la description ci-après, ce trou, en particulier en combinaison avec l'orifice de la deuxième patte, permet de stabiliser l'armature provisoirement en per-opératoire à l'aide de vis temporaires engagées dans ce trou et/ou ledit orifice.

Un deuxième aspect de l'invention est un insert, destiné à séparer une armature de fond de cotyle de la cupule prothétique acétabulaire à implanter, ladite armature de fond de cotyle étant munie sur sa face interne, destinée à être disposée en regard de ladite cupule prothétique acétabulaire, d'au moins un évidement, caractérisé en ledit insert est apte à se déformer d'une position élargie sans contrainte à une position rétrécie sous contrainte, ledit insert étant destiné à être engagé dans ledit évidement lorsqu'il est dans sa position rétrécie.

Ainsi, un autre aspect de l'invention concerne un ensemble comprenant une armature telle que décrite ci-dessus, au moins une desdites pattes étant munie d'au moins un évidement sur sa face interne, ledit ensemble étant caractérisé en ce qu'il comprend en outre au moins un insert apte à se déformer d'une position élargie sans contrainte à une position rétrécie sous contrainte, ledit insert étant destiné à être engagé dans ledit évidement lorsqu'il est dans sa position rétrécie.

Dans une forme de réalisation de l'invention, ledit insert consiste en une seule pièce de forme de révolution sensiblement cylindrique creuse comportant une fente selon une génératrice parallèle à son axe de révolution. Ce dispositif ou insert est introduit en position rétrécie par l'effet de la fente et de son élasticité dans les orifices ou évidements prévus dans les pattes de ladite armature en croix. Cette invention ne nécessite qu'une seule taille utilisable dans un ou plusieurs trous ou évidements selon les besoins opératoires. En outre, ce dispositif ou insert est d'un coût très réduit. Par ailleurs, l'utilisation d'un ou plusieurs inserts selon l'invention pour séparer une armature de fond de cotyle de l'implant cotyloïdien destiné à être reçu au sein de ladite armature permet de réduire la quantité de corps étranger implanté dans le corps du patient.

Un autre aspect de l'invention concerne un instrument chirurgical de placement d'une armature de fond de cotyle au sein d'une cavité acétabulaire, ladite armature de fond de cotyle étant munie sur sa face interne d'au moins un évidement, ledit instrument comprenant au moins une poignée et au moins une tige allongée, la tige allongée comprenant à son extrémité distale au moins un têton de premier positionnement de ladite tige allongée par rapport à l'axe de révolution correspondant à l'axe de la demi-sphère de la cavité acétabulaire, lorsque ledit têton est engagé dans ledit évidement, caractérisé en ce que ledit instrument comprend en outre des moyens de repositionnement de ladite tige allongée par rapport audit axe de révolution, lesdits moyens de repositionnement étant aptes à modifier le positionnement de ladite tige allongée par rapport audit axe de révolution sans désengager ledit têton dudit évidement.

Dans la présente demande, l'extrémité distale d'un composant ou d'un dispositif doit être comprise comme signifiant l'extrémité la plus éloignée de la main de l'utilisateur et l'extrémité proximale doit être comprise comme signifiant l'extrémité la plus proche de la main de l'utilisateur.

Dans la présente demande, on entend par « face interne » d'une armature de fond de cotyle, la face de cette armature destinée à être disposée en regard de la cupule de l'implant cotyloïdien destiné à être reçu au sein de ladite armature, et par « face externe » la face de cette armature destinée à être disposée en regard de l'os du bassin.

Ainsi, un autre aspect de l'invention est un ensemble comprenant une armature telle que décrite ci-dessus, ladite armature de fond de cotyle étant munie sur sa face interne d'au moins un évidement, ledit ensemble étant caractérisé en ce qu'il comprend en outre au moins un instrument chirurgical de placement de ladite armature au sein d'une cavité acétabulaire ledit instrument comprenant au moins une poignée et au moins une tige allongée, la tige allongée comprenant à son extrémité distale au moins un têton de premier positionnement de ladite tige allongée par rapport à l'axe de révolution correspondant à l'axe de la demi-sphère de la cavité acétabulaire, lorsque ledit têton est engagé dans ledit évidement, ledit instrument comprenant en outre des moyens de repositionnement de ladite tige allongée par rapport audit axe de révolution, lesdits moyens de repositionnement étant aptes à modifier le positionnement de ladite tige allongée par rapport audit axe de révolution sans désengager ledit têton dudit évidement.

Dans une forme de réalisation de l'invention, lesdits moyens de repositionnement comprennent i) au moins une plaque solidaire dudit téton, ladite plaque étant munie d'au moins un ergot, et ii) au moins une coque solidaire de l'extrémité distale de ladite tige allongée, ladite coque comprenant à son extrémité distale une semelle munie sur sa face distale d'une pluralité de logements disposés selon un cercle, ladite plaque et ladite coque étant alignées selon l'axe de révolution dudit cercle et étant couplées l'une à l'autre par des moyens de rappel élastique, lesdits moyens de rappel élastique étant aptes à se déformer d'une position sous contrainte élevée, dans laquelle ladite plaque est mobile en rotation par rapport à ladite coque autour de l'axe de révolution dudit cercle, à une position de moindre contrainte, dans laquelle ledit ergot est engagé dans au moins un logement de ladite pluralité de logements, ladite plaque étant alors bloquée en rotation vis-à-vis de ladite coque.

Dans une forme de réalisation de l'invention, lesdits moyens de rappel élastique comprennent au moins un ressort hélicoïdal logé au sein d'un alésage cylindrique central traversant ladite coque et ladite semelle, ladite plaque étant munie d'un orifice central fileté aligné sur ledit alésage cylindrique, et une vis prenant appui sur ledit ressort hélicoïdal étant reçue au sein dudit alésage cylindrique central, la partie filetée de la vis étant engagée dans l'orifice central fileté de ladite plaque, ledit ressort hélicoïdal et ladite vis réalisant ainsi le couplage par des moyens de rappel élastique de ladite coque à ladite plaque.

Dans une forme de réalisation de l'invention, ladite armature comprenant au moins deux évidements, ladite plaque comprend au moins deux têtons, chaque têton étant destiné à être engagé dans un évidement.

Dans une forme de réalisation de l'invention, ladite plaque comprend au moins deux ergots, de préférence diamétralement opposés, chaque ergot étant destiné à être engagé dans un logement de ladite pluralité de logements. L'équilibre et la stabilité de l'instrument chirurgical sont ainsi assurés pendant le déroulement de l'opération de placement de l'armature au sein de la cavité acétabulaire.

Dans une forme de réalisation de l'invention, l'armature comprenant un alésage central fileté, la partie filetée de ladite vis est en outre engagée dans ledit alésage central fileté, réalisant ainsi la solidarisation dudit instrument chirurgical à ladite armature.

Un aspect de l'invention est donc un dispositif comprenant l'armature telle que décrite ci-dessus et un instrument spécifique destiné à faciliter la mise en place de ladite armature, ledit instrument étant caractérisé par sa forme décalée et ses moyens de repositionnement permettant le positionnement de ladite armature tout en laissant la possibilité à l'opérateur de choisir l'orientation optimale de cet instrument par rapport à l'armature, afin de dégager l'accès à la cavité acétabulaire pour la mise en place de vis éventuelles.

De préférence, l'instrument chirurgical selon l'invention est utile non seulement pour le positionnement de l'armature dans la cavité acétabulaire mais permet aussi à l'opérateur de maintenir ladite armature pendant le cintrage de la plaque ovalaire et galbée.

Ainsi, un autre aspect de l'invention est une méthode de fixation d'une armature de fond de cotyle au sein d'une cavité acétabulaire de l'os du bassin, comprenant les étapes suivantes :
- A) Le chirurgien dispose d'une armature de fond de cotyle selon l'art antérieur ou telle que décrite ci-dessus, et d'un instrument chirurgical tel que décrit ci-dessus,
- B) Eventuellement, si nécessaire et si le chirurgien dispose d'une armature selon la présente invention, le chirurgien casse l'extrémité distale d'une ou de plusieurs pattes autocassables de ladite armature afin d'adapter la structure de ladite armature au défaut de l'os du bassin à traiter,
- C) Le chirurgien dispose ensuite l'armature au sein de la cavité acétabulaire en logeant la pointe recourbée de la première patte de ladite armature dans le trou obturateur de l'os du bassin,
- D) Le chirurgien fixe de façon temporaire l'armature à l'os du bassin par le moyen de vis qu'il visse dans l'os du bassin à travers des orifices appropriés ménagés sur ladite armature,
- E) Le chirurgien vient placer les têtons de l'instrument chirurgical dans les évidements prévus à cet effet sur la face interne de ladite armature, réalisant ainsi un premier positionnement de la tige allongée dudit instrument par rapport à l'axe de révolution de la demi-sphère correspondant à la cavité acétabulaire,
- F) Le chirurgien solidarise ensuite l'instrument chirurgical à ladite armature de fond de cotyle, par exemple en vissant la vis de l'instrument chirurgical dans l'alésage central fileté de ladite armature,
- G) Eventuellement, si l'orientation de la tige allongée par rapport audit axe de révolution ne convient pas au chirurgien, ce dernier repositionne la tige allongée à l'aide des moyens de repositionnement dudit instrument, en faisant tourner ladite tige autour de l'axe de révolution desdits moyens de repositionnement, autant de fois que nécessaire, jusqu'à obtenir l'orientation de la tige allongée qu'il juge optimale,
- H) Le chirurgien saisit alors la plaque ovalaire et galbée par un outil approprié et, tout en maintenant l'armature fermement à l'aide dudit instrument chirurgical avec une main, exerce avec l'autre main et au moyen dudit outil approprié un mouvement de couple sur ladite plaque ovalaire et galbée afin de tordre la deuxième patte de ladite armature et ainsi plaquer ladite plaque ovalaire et galbée sur la partie supérieure de l'os du bassin,
- I) Le chrurgien cintre et fixe alors ladite plaque ovalaire et galbée sur la partie supérieure de l'os du bassin à l'aide de vis vissées dans ledit os à travers les orifices de la plaque ovalaire et galbée,
- J) Le chirurgien désolidarise alors l'instrument chirurgical de ladite armature et retire les vis de fixation temporaire,
- K) Du ciment est ensuite coulé au fond de la cavité acétabulaire entre l'armature de fond de cotyle et l'os pour réaliser la fixation définitive de ladite armature au sein de la cavité acétabulaire.

La présente invention va maintenant être décrite plus en détail à l'aide de la description suivante et des figures annexées dans lesquelles :
- les Figures 1A et 1B sont des vues détaillées en perspective de l'armature selon l'invention, respectivement avant et après retrait d'une partie d'extrémité libre d'une patte autocassable,
- la Figure 2 est une vue en perspective d'un insert ou « spacer » selon l'invention.
- les Figures 3A et 3B sont des vues en coupe de l'instrument chirurgical selon l'invention lorsqu'il est vissé sur l'armature selon l'invention, dans la position de moindre contrainte, respectivement de contrainte élevée, des moyens de rappel élastique des moyens de repositionnement,
- la figure 4A est une vue de dessus de la plaque de l'instrument chirurgical de la figure 3A,
- la figure 4B est une vue de dessous de la plaque de l'instrument chirurgical de la figure 3A,
- la figure 5 est une vue de dessous de la semelle de la coque de l'instrument chirurgical de la figure 3A,
- les figures 6A et 6B sont des vues en perspective de l'instrument chirurgical de l'invention chargé de l'armature selon l'invention suivant deux orientations différentes, c'est-à-dire respectivement avant et après repositionnement de la tige allongée par rapport à l'axe de révolution de la demi sphère correspondant à la cavité acétabulaire.

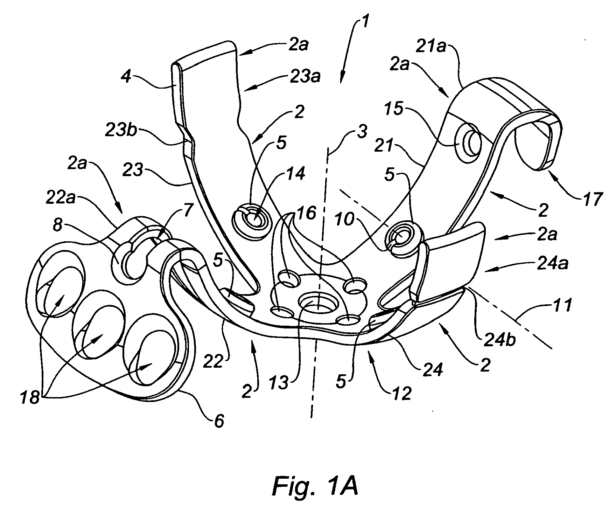

En référence à la figure 1A, l'armature 12 de la présente invention comprend une croix galbée 1 comportant au moins quatre pattes 2 concentriques réparties autour de l'axe de révolution 3 correspondant à l'axe de la demi sphère de la cavité acétabulaire (non représentée). Comme il apparaît clairement sur la figure 1A, les quatre pattes 2 forment les quatre branches de la croix 1 : ainsi, chaque patte 2 présente une extrémité fixe rejoignant les extrémités fixes des trois autres pattes au centre de la croix 1 et une extrémité libre 2a. Par ailleurs, ces quatre pattes 2 présentent chacune une forme longitudinale légèrement arquée, réalisant ainsi le galbe de la croix 1. La croix galbée 1 ainsi réalisée définit une armature 12 délimitant une demi-sphère et destinée à épouser sensiblement la forme d'une cavité acétabulaire de l'os du bassin, afin d'être fixée à cet os, et à recevoir en son sein un implant cotyloïdien tel qu'une prothèse de hanche, en particulier la cupule d'un tel implant.

La position desdites pattes 2 peut être équidistante ou non. Sur l'exemple représenté à la figure 1A, chaque patte 2 est espacée de la patte 2 adjacente par un angle sensiblement égal à 90°.

Parmi les quatre pattes 2 représentées sur la figure 1A, une première patte 21 comporte à son extrémité libre 21 a une pointe recourbée 17 destinée à être logée dans le trou obturateur (non représenté) du bassin lorsque l'armature 12 est implantée. Cette première patte 21 comporte en outre dans la région de son extrémité libre 21a un trou 15 de forme sensiblement circulaire, dont la fonction sera expliquée plus loin.

Une deuxième patte (2, 22) destinée à être positionnée dans la face antéro-externe de la cavité acétabulaire lorsque l'armature 12 est implantée, comporte à son extrémité libre 22a une plaque 6 sensiblement ovalaire et galbée, destinée à être fixée au support osseux : pour ce faire, la plaque 6 est apte à recevoir des vis de fixation osseuse (non représentées) au sein de logements traversants 18, au nombre de trois sur l'exemple représenté, percés dans ladite plaque 6.

Cette deuxième patte (2, 22) est destinée à s'adapter au plan osseux. Pour ce faire, elle est munie d'une fente longitudinale 7 permettant de la modeler au support osseux.

Dans les armatures de l'art antérieur, la fente précitée tend à fragiliser la patte qui la comporte. Ainsi, dans les armatures de l'art antérieur, il peut arriver que la patte se casse lorsqu'on la tord pour la modeler au support osseux. L'armature 12 selon l'invention est donc améliorée par la présence d'un ou plusieurs orifices 8 de forme sensiblement circulaire situé(s) dans le prolongement de ladite fente longitudinale 7 au niveau de la plaque 6 ovalaire et galbée et permettant de dissiper les contraintes lors du modelage de la deuxième patte (2, 22) et d'éviter la rupture de cette dernière. En effet, lorsque l'armature 12 est mise en place dans la cavité acétabulaire, la deuxième patte 22 est tordue afin que la plaque 6 ovalaire et galbée puisse venir se plaquer puis être fixée sur le support osseux. La présence de l'orifice 8 de forme sensiblement circulaire dans le prolongement de la fente longitudinale 7 permet de tordre, donc de modeler, la deuxième patte 22 sans la casser.

Comme il apparaît sur la figure 1A, la première patte 21 et la deuxième patte 22 sont opposées l'une à l'autre, c'est-à-dire qu'elles constituent deux branches opposées de la croix galbée 1 et qu'elles sont espacées d'un angle de 180°.

Le ou les orifices 8 de forme sensiblement circulaire de la deuxième patte 22, ainsi que le trou ou orifice additionnel 15, situé sur la première patte (2, 21) opposée à la deuxième patte 22 comportant la plaque 6, permettent une seconde fonction, à savoir la possibilité de stabiliser provisoirement en per-opératoire l'armature 12 à l'aide de vis temporaires sans limiter la possibilité de mettre l'armature 12 en tension car les vis temporaires situées dans le ou les orifices 8 peuvent glisser librement dans la fente longitudinale 7.

Par ailleurs, la croix galbée 1 comporte en son fond, c'est-à-dire en son centre où se rejoignent les extrémités fixes des quatre pattes 2, un ou plusieurs (quatre dans l'exemple représenté) orifices 16 supplémentaires. L'ensemble de ces orifices ou trous (8,15,16) de la première patte 21, de la deuxième patte 22 et du fond de la croix 1, une fois libérés après implantation, assurent la pénétration du ciment destiné à remplir l'espace entre l'armature 12 et l'os du bassin, assurant un meilleur ancrage entre le ciment et ladite armature 12. Sur l'exemple représenté, la croix galbée 1 comporte également en son fond un alésage central fileté 13 dont la fonction sera expliquée plus loin.

L'extrémité libre 17 de la patte 2 comportant le trou 15 se termine en forme de pointe destinée à être logée dans le trou obturateur, rendant ainsi l'armature 12 plus anatomique.

L'obliquité de la cavité acétabulaire favorise l'emploi d'armature en croix asymétrique au détriment du nombre de pièces et de leur coût étant donné qu'il faut un grand nombre de tailles pour s'adapter au diamètre de la cavité acétabulaire. C'est pourquoi l'armature 12 selon l'invention est améliorée par l'adoption de pattes autocassables 4, situées aux extrémités libres de certaines des pattes 2, dont le but est par la rupture d'une extrémité d'une des pattes 2 de récréer l'asymétrie précitée et donc d'éviter un surcroît de tailles. Cet auto-cassage se fait préalablement à l'implantation en fonction du côté sélectionné.

A cet effet, en référence à la figure 1A, la croix galbée 1 comporte une troisième patte 23 et une quatrième patte 24, formant avec la première et la deuxième pattes (21, 22) les quatre branches de la croix 1. Sur l'exemple représenté, la troisième patte 23 et la quatrième patte 24 sont opposées l'une à l'autre, c'est-à-dire séparées l'une de l'autre par un angle de 180°. Comme il apparaît clairement sur la figure 1A, la troisième patte 23 et la quatrième patte 24 comportent chacune dans la région de son extrémité libre 23a (respectivement 24a), une partie rétrécie 23b (respectivement 24b) autorisant à son endroit la rupture de ladite patte 23 (respectivement 24). Ainsi, la troisième patte 23 et la quatrième patte 24 sont autocassables : en d'autres termes, la partie d'extrémité libre (23a, 24a) de chacune de ces pattes (23, 24) peut être détachée du reste du corps de ladite patte (23, 24) de façon aisée par le chirurgien, sur le site opératoire, au moment de l'opération. Le chirurgien peut s'aider éventuellement d'une pince (non représentée) avec laquelle il saisit la partie d'extrémité libre (23a, 24a) et exerce une force de levier au niveau de la partie rétrécie (23b, 24b), détachant ainsi la partie d'extrémité libre (23a, 23b) du reste du corps de la patte (23, 24).

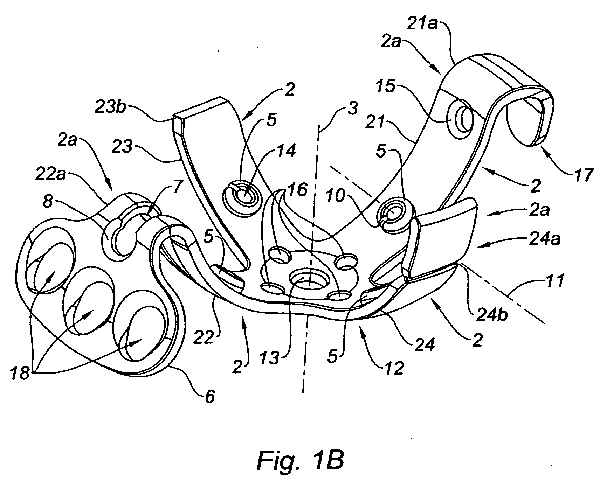

Sur la figure 1B est représentée l'armature 12 de la figure 1A une fois que le chirurgien a retiré la partie d'extrémité libre 23a de la troisième patte 23. Une telle armature 12 est asymétrique et est donc parfaitement adaptée pour une implantation au sein d'une cavité acétabulaire.

En référence à la figure 1A, chacune des pattes (2, 21, 22, 23, 24) est munie d'un insert 5 (ou spacer) faisant saillie de la surface interne de chaque patte, la surface interne de chaque patte étant définie comme étant la surface de la patte destinée à être disposée en regard de l'implant cotyloïdien.

Cet insert 5 est un dispositif, aussi appelé « spacer », permettant de séparer l'armature 12 en croix et la cupule prothétique acétabulaire (non représentée) à implanter. Un tel insert 5 est destiné à limiter, voire éviter, toute métalose ou réaction par contact et/ou friction entre l'armature 12 de fond de cotyle et la cupule de l'implant cotyloïdien que cette dernière est destinée à recevoir.

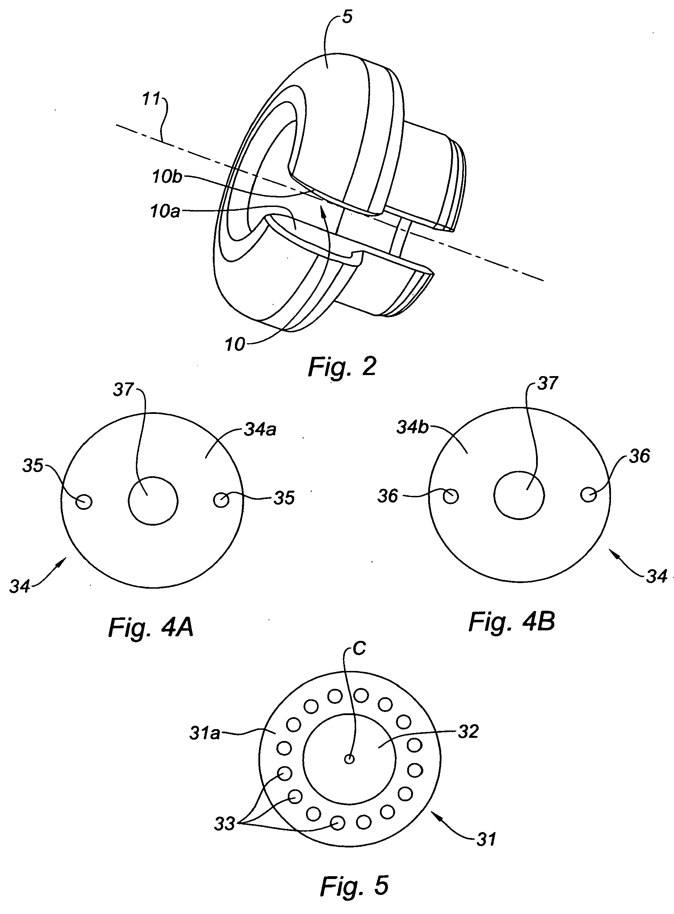

En référence à la figure 2, le « spacer » ou insert 5 fait l'objet également d'une amélioration et d'une simplification importante puisqu'il consiste en une seule pièce 5 de forme de révolution sensiblement cylindrique creuse comportant une fente 10 selon une génératrice parallèle à son axe de révolution 11. Cet insert 5 est à apte se déformer d'une position élargie sans contrainte, telle que représentée sur la figure 2, à une position rétrécie sous contrainte, par exemple lorsqu'une force exercée sur ledit insert 5 tend à rapprocher l'un de l'autre les deux bords opposés 10a et 10 b de la fente 10 : en particulier ledit insert 5 est destiné à être engagé dans un évidement 14 ménagé sur au moins l'une des pattes (2, 21, 22, 23, 24) de l'armature 12 selon l'invention, lorsqu'il est dans sa position rétrécie, comme montré sur les figures 1A et 1B où chacune des pattes (2, 21, 22, 23, 24) est munie d'un tel insert 5. Ce dispositif ou insert 5 est introduit en position rétrécie par l'effet de la fente 10 et de son élasticité dans les orifices ou évidements 14 prévus dans les pattes 2 de ladite armature 12 comme montré sur la figure 1A. Le diamètre interne de l'évidement 14 étant inférieur au diamètre correspondant au diamètre externe de la partie de l'insert 5 engagée dans l'évidement 14 lorsque ledit insert est dans sa position élargie, l'insert 5 est maintenu fermement engagé dans ledit évidement 14 par friction.

Dans une forme de réalisation de l'invention, l'insert 5 est en matière plastique présentant une certaine élasticité, comme par exemple du polyméthacrylate de méthyle (PMMA).

Comme il apparaît des figures 1A et 1B, une partie de l'insert 5 fait saillie de la surface interne de la patte délimitant ainsi un bossage empêchant la cupule (non représentée) de l'implant cotyloïdien destiné à être reçu dans l'armature 12 d'entrer en contact avec ladite armature 12. De préférence, chaque patte (2, 21, 22, 23, 24) de l'armature 12 est munie d'un tel évidement 14 dans lequel est engagé un tel insert 5.

Un tel insert 5 selon l'invention simplifie grandement les armatures de l'art antérieur en ne nécessitant qu'une seule taille utilisable dans un ou plusieurs trous ou évidements 14 selon les besoins opératoires. En outre, ce dispositif ou insert simplifié est d'un coût très réduit par rapport à un système tel que décrit dans FR 2 842 096 A1. Par ailleurs, l'utilisation d'un ou plusieurs inserts 5 selon l'invention permet de réduire la quantité de corps étranger implanté dans le corps du patient. La simplification du « spacer » ou insert 5 permet d'adapter, dans l'orifice ou alésage central 13 situé au pôle de l'armature 12, ainsi libéré, un ou des instruments facilitant la mise en place per-opératoire, comme il sera expliqué ci-après.

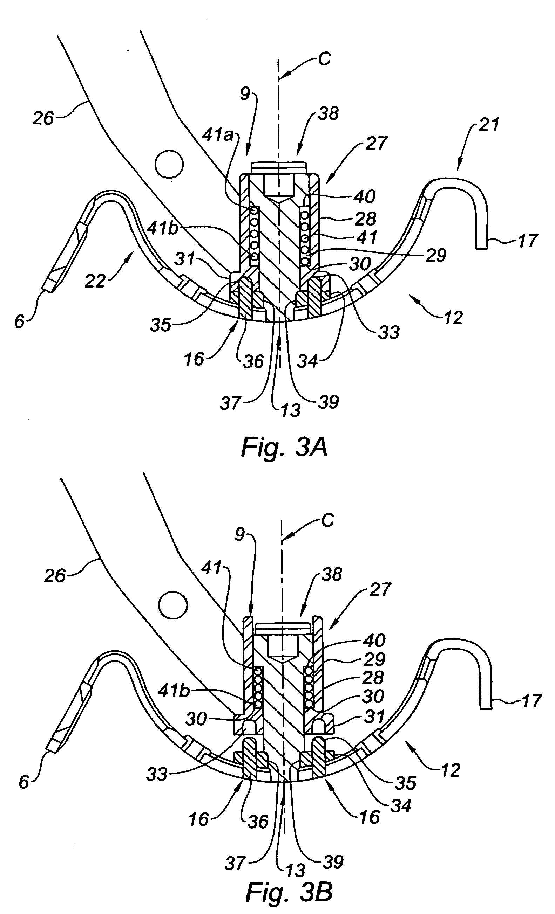

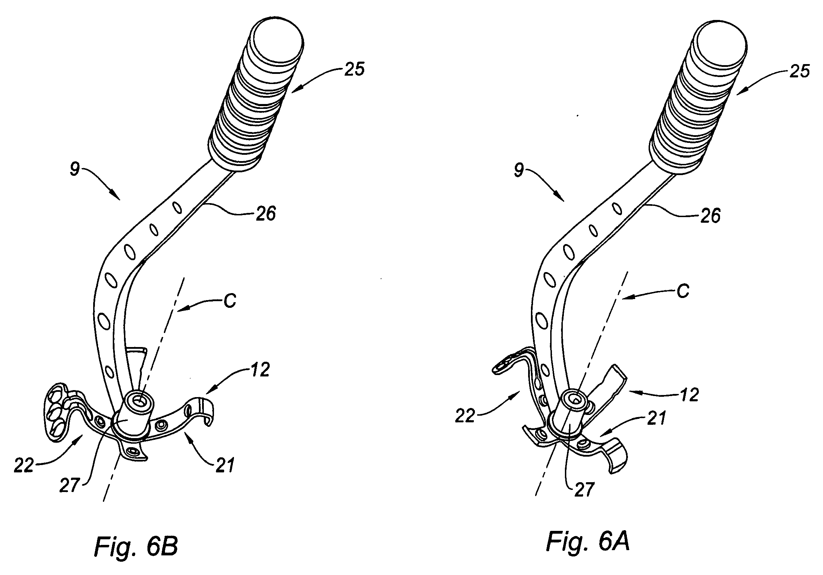

En référence aux figures 3A, 3B, 6A et 6B, un instrument chirurgical 9 selon l'invention pour placer une armature de fond de cotyle au sein d'une cavité acétabulaire est représenté vissé sur une armature 12 telle que décrite ci-dessus.

Bien que l'instrument chirurgical 9 selon l'invention et son fonctionnement soient illustrés en combinaison avec une armature de fond de cotyle selon la présente invention, il est à noter que l'instrument chirurgical 9 selon l'invention peut être utilisé avec une armature de fond de cotyle de l'art antérieur, en particulier avec des armatures de fond de cotyle ne possédant pas de pattes autocassables.

L'instrument chirurgical 9 comprend une poignée 25 et une tige allongée 26 : comme il apparaît sur ces figures, la tige allongée 26 présente substantiellement un axe longitudinal et peut être légèrement courbée. Une telle courbure facilite l'opération de placement de l'armature 12 au sein de la cavité acétabulaire par le chirurgien. La tige allongée 26 est munie à son extrémité distale d'une coque 27, solidaire de la tige allongée 26. La coque 27 présente la forme d'un fourreau sensiblement cylindrique 28 traversé par un alésage cylindrique central 29. Le fourreau 28 est muni sur la paroi interne de sa région distale d'un rebord annulaire 30.

La coque 27 est munie à son extrémité distale d'une semelle 31. En référence à la figure 5 qui est une vue de dessous de la semelle 31, cette dernière est également traversée par un alésage 32. Toujours en référence à la figure 5, la face distale 31a de la semelle 31 est munie d'une pluralité de logements 33 disposés selon un cercle présentant un axe de révolution C.

En référence aux figures 3A et 3B, l'instrument chirurgical 9 selon l'invention comprend en outre une plaque 34 de forme sensiblement circulaire, alignée sur la coque 27 selon l'axe de révolution C. En référence à la figure 4A, qui est une vue de dessus de la plaque 34, la face proximale 34a de la plaque 34 est munie de deux ergots 35 faisant saillie de cette face proximale 34a et diamétralement opposés. En référence à la figure 4B, qui est une vue de dessous de la plaque 34, la face distale 34b de la plaque 34 est munie de deux têtons 36 faisant saillie de cette face distale 34b et diamétralement opposés. En référence aux figures 4A et 4B, la plaque 34 est traversée en son centre par un orifice central fileté 37.

Comme il apparaît sur la figure 3A, un têton 36 et un ergot 35 peuvent constituer une seule pièce cylindrique traversant la plaque 34 et faisant saillie de part et d'autre de cette plaque. Alternativement, dans une forme de réalisation de l'invention non représentée, chaque têton est indépendant de chaque ergot.

En référence aux figures 3A et 3B, l'instrument chirurgical 9 est en outre muni d'une vis 38 reçue au sein de l'alésage cylindrique central 29 du fourreau 28 de la coque 27. Comme il apparaît sur ces figures, l'alésage cylindrique central 29 du fourreau 28, l'alésage 32 de la semelle 31 et l'orifice central fileté 37 étant alignés sur l'axe C, la vis 38 traverse à la fois la coque 27 et sa semelle 31 ainsi que la plaque 34. Par ailleurs, l'extrémité distale de la vis 38 est munie d'un filetage externe 39 apte à coopérer avec l'orifice central fileté 37 de la plaque 34 de telle sorte que la vis 38 est vissée dans ladite plaque 34.

La vis 38 est munie dans sa région proximale d'un décrochement annulaire externe 40. Un ressort hélicoïdal 41 est disposé entre la paroi interne du fourreau 28 et la vis 38. L'extrémité proximale 41 a du ressort 41 prend appui sur la face distale du décrochement annulaire externe 40 de la vis 38 et l'extrémité distale 41b du ressort 41 prend appui sur la face proximale du rebord annulaire 30 du fourreau 28. Ainsi, comme il apparaîtra de la description des figures 3A et 3B ci-dessous, la vis 38 est déplaçable en translation par rapport à la coque 27. La coque 27 et la plaque 34 sont ainsi couplées l'une à l'autre au moyen du ressort hélicoïdal 41 et de la vis 38 qui réalisent ainsi un couplage par des moyens de rappel élastique.

En référence aux figures 3A, 3B, 6A et 6B, une étape de repositionnement de l'instrument chirurgical 9 par rapport à l'axe de révolution de la demi sphère d'une cavité acétabulaire va maintenant être décrite. Pour des raisons de clarté, la cavité acétabulaire n'est pas représentée sur ces figures et il sera entendu que l'axe de révolution de la demi sphère d'une cavité acétabulaire correspond à l'axe C représenté sur ces figures.

Sur la figure 3A est représenté l'instrument chirurgical 9 vissé à une armature 12 selon la figure 1 de la présente demande dans un plan de coupe passant par la première patte 21 et la deuxième patte 22 de l'armature 12. La figure 3A correspond à la vue en perspective 6A sur laquelle il apparaît que la tige allongée 26 est substantiellement incluse dans ce plan de coupe passant par la première patte 21 et la deuxième patte 22 de l'armature 12. L'instrument chirurgical 9 est ainsi dans une position de premier positionnement : dans cette position, comme représenté sur la figure 3A, les têtons 36 sont engagés dans les évidements ou orifices supplémentaires 16 de l'armature 12 : par ailleurs, le chirurgien a vissé la vis 38 dans l'alésage central fileté 13 situé sur le fond de l'armature 12 au moyen du filetage 39 de la vis 38 qui a coopéré avec ledit alésage central fileté 13. Dans cette position, l'instrument chirurgical 9 est donc solidarisé avec l'armature 12. Dans cette position représentée sur la figure 3A, le ressort hélicoïdal 41 est en position de moindre contrainte, les ergots 35 sont engagés dans deux logements 33 diamétralement opposés de la semelle 31, et la coque 27, donc la tige allongée 26, est bloquée en rotation par rapport à la plaque 35, elle-même fixe vis-à-vis de l'armature 12, de par l'engagement des têtons 36 dans les orifices 16 de l'armature.

Dans l'orientation de la tige 26 telle que représentée aux figures 3A et 6A, le chirurgien ne peut accéder aisément à la plaque ovalaire et galbée 6 de l'armature 12 dans le but de la saisir avec une pince et de tordre la deuxième patte 22 pour plaquer cette plaque ovalaire et galbée 6 contre l'os du bassin (non représenté). Il est donc nécessaire de repositionner l'orientation de la tige 26 par rapport à l'armature 12 et donc par rapport à l'axe C.

Pour cela, le chirurgien, au moyen d'un outil adapté, exerce une pression distale sur l'extrémité proximale de la vis 38 tout en tirant à lui, c'est-à-dire dans le sens proximal, la tige allongée 26 de l'instrument chirurgical 9. Auparavant, le chirurgien aura fixé, de façon temporaire, l'armature 12 à l'os du bassin aux moyens de vis temporaires introduites dans les orifices 8 et/ou trou 15 de l'armature 12 (voir figure 1).

Ainsi, lorsque le chirurgien tire vers lui la tige allongée 26 comme décrit ci-dessus, le ressort hélicoïdal 41 est amené à passer de sa position de moindre contrainte, telle que représentée à la figure 3A à sa position de contrainte élevée, telle que représentée à la figure 3B. Ce faisant, la coque 27 et la semelle 31 se déplacent dans le sens proximal par rapport à la plaque 34 et les ergots 35 se désengagent des logements 33, comme montré sur la figure 3B. Tout en maintenant le ressort hélicoïdal 41 sous contrainte élevée, le chirurgien a alors tout loisir de faire pivoter la coque 27 et donc la tige allongée 26 autour de l'axe C, par rapport à la plaque 34, qui reste immobile vis-à-vis de l'armature 12 du fait de l'engagement des têtons 36 dans les orifices 16. Le chirurgien fait ainsi tourner la coque 27 et la tige allongée 26 jusqu' obtenir l'orientation de la tige allongée 26 qu'il juge optimale pour la suite des opérations. Lorsque cette orientation optimale est atteinte, comme par exemple celle montrée à la figure 6B, le chirurgien laisse la tige allongée 26 revenir dans le sens distal par rappel élastique du ressort hélicoïdal 41 qui tend à revenir vers sa position de moindre contrainte. Lors de ce rappel élastique, les ergots 35 sont amenés à s'engager dans deux nouveaux logements 33 diamétralement opposés de la pluralité de logements 33 disposés en cercle de la semelle 31, ces nouveaux logements étant éloignés des logements initiaux 33 représentés sur la figure 3A par un angle correspondant à l'angle dont le chirurgien a fait tourner la coque 27, et donc la semelle 31, par rapport à la plaque 34. Ainsi, la coque 27 est de nouveau bloquée en rotation par rapport à la plaque 34 et a été repositionnée par le chirurgien de façon à disposer de l'orientation optimale de la tige allongée 26 par rapport à l'axe C pour pouvoir permettre au chirurgien de saisir la plaque ovalaire et galbée 6 de l'armature 12.

Ainsi, l'instrument 9 est remarquable en ce qu'il permet, par sa forme décalée et par ses moyens de repositionnement, le positionnement de ladite armature 12 tout en laissant la possibilité à l'opérateur de choisir l'orientation de cet instrument 9 par rapport à l'armature 12, de façon optimale, afin de dégager l'accès à la cavité acétabulaire pour la mise en place de vis éventuelles. Cet instrument 9 est utile non seulement pour le positionnement de la croix 1 dans la cavité acétabulaire mais permet aussi à l'opérateur de maintenir ladite armature 12 pendant le cintrage de la plaque ovalaire et galbée 6.

## Revendications

1. Armature (12) pour fond de cotyle comprenant une croix galbée (1) comportant au moins quatre pattes (2) concentriques réparties autour de l'axe de révolution (3) correspondant à l'axe de la demi sphère de la cavité acétabulaire et, parmi lesdites quatre pattes (2), au moins une première patte (21) comportant à son extrémité libre (21a) une pointe recourbée (17) destinée à être logée dans le trou obturateur du bassin lorsque l'armature (12) est implantée, au moins une deuxième patte (22), destinée à être positionnée dans la face antéro-externe de la cavité acétabulaire lorsque l'armature (12) est implantée, comportant à son extrémité libre (22a) une plaque (6) sensiblement ovalaire et galbée, destinée à être fixée au support osseux, ladite deuxième patte (22) étant munie d'une fente longitudinale (7), **caractérisée en ce que** ladite fente se terminant, au niveau de ladite plaque (6) ovalaire et galbée, par au moins un orifice (8) de forme sensiblement circulaire et qu'au moins une des troisième et quatrième pattes étant autocassable.

2. Armature (12) selon la revendication 1, **caractérisée en ce que** ladite première patte (21) et ladite deuxième patte (22) sont opposées l'une à l'autre et lesdites troisième (23) et quatrième (24) pattes sont autocassables.

3. Armature (12) selon l'une des revendications 1 à 2, **caractérisée en ce que** chacune de la ou desdites patte(s) autocassable(s) (23, 24) comporte dans la région de son extrémité libre (23a, 24a) une partie rétrécie (23b, 24b) autorisant à son endroit la rupture de ladite patte (23, 24).

4. Armature (12) selon l'une des revendications 1 à 3, **caractérisée en ce que** ladite première patte (21) comporte dans la région de son extrémité libre (21a) un trou (15) de forme sensiblement circulaire.

5. Ensemble comprenant une armature (12) selon l'une quelconque des revendications 1 à 4, au moins une desdites pattes étant munie d'au moins un évidement (14) sur sa face interne, ledit ensemble comprenant en outre au moins un insert (5) apte à se déformer d'une position élargie sans contrainte à une position rétrécie sous contrainte, ledit insert (5) étant destiné à être engagé dans ledit évidement (14) lorsqu'il est dans sa position rétrécie.

6. Ensemble selon la revendication 5, **caractérisé en ce que** ledit insert (5) consiste en une seule pièce de forme de révolution, sensiblement cylindrique creuse comportant une fente (10) selon une génératrice parallèle à son axe de révolution (11).

7. Ensemble selon la revendication 5 ou 6, **caractérisé en ce que** chaque patte (2, 21, 22, 23, 24) de l'armature (12) est munie d'un dit évidement (14) dans lequel est engagé un dit insert (5).

8. Ensemble comprenant une armature (12) selon l'une des revendications 1 à 4, ladite armature étant munie sur sa face interne d'au moins un évidement (16), ledit ensemble comprenant en outre au moins un instrument chirurgical (9) de placement de ladite armature (12) au sein d'une cavité acétabulaire, ledit instrument (9) comprenant au moins une poignée (25) et au moins une tige allongée (26), la tige allongée (26) comprenant à son extrémité distale au moins un têton (36) de premier positionnement de ladite tige allongée (26) par rapport à l'axe de révolution C correspondant à l'axe de la demi-sphère de la cavité acétabulaire, lorsque ledit têton (36) est engagé dans ledit évidement (16), ledit instrument (9) comprenant en outre des moyens de repositionnement (27, 31, 33, 34, 35, 41) de ladite tige allongée (26) par rapport audit axe de révolution C, lesdits moyens de repositionnement étant aptes à modifier le positionnement de ladite tige allongée (26) par rapport audit axe de révolution C sans désengager ledit têton (36) dudit évidement (16).

9. Ensemble selon la revendication 8, **caractérisé en ce que** lesdits moyens de repositionnement comprennent i) au moins une plaque (34) solidaire dudit têton (36), ladite plaque étant munie d'au moins un ergot (35), et ii) au moins une coque (27) solidaire de l'extrémité distale de ladite tige allongée (26), ladite coque (27) comprenant à son extrémité distale une semelle (31) munie sur sa face distale (31a) d'une pluralité de logements (33) disposés selon un cercle, ladite plaque (34) et ladite coque (27) étant alignées selon l'axe de révolution dudit cercle et étant couplées l'une à l'autre par des moyens de rappel élastique (41), lesdits moyens de rappel élastique étant aptes à se déformer d'une position sous contrainte élevée, dans laquelle ladite plaque (34) est mobile en rotation par rapport à ladite coque (27) autour de l'axe de révolution dudit cercle, à une position de moindre contrainte, dans laquelle ledit ergot (35) est engagé dans au moins un logement (33) de ladite pluralité de logements, ladite plaque (34) étant alors bloquée en rotation vis-à-vis de ladite coque (27).

10. Ensemble selon la revendication 9, **caractérisé en ce que** lesdits moyens de rappel élastique comprennent au moins un ressort hélicoïdal (41) logé au sein d'un alésage cylindrique central (29, 32) traversant ladite coque (27) et ladite semelle (31), ladite plaque (34) étant munie d'un orifice central fileté (37) aligné sur ledit alésage cylindrique (29, 32), une vis (38) prenant appui sur ledit ressort hélicoïdal (41) étant reçue au sein dudit alésage cylindrique central (29, 32), la partie filetée (39) de la vis (38) étant engagée dans l'orifice central fileté (37) de ladite plaque (34), ledit ressort hélicoïdal (41) et ladite vis (38) réalisant ainsi le couplage par des moyens de rappel élastique de ladite coque (27) à ladite plaque (34).

11. Ensemble selon l'une quelconque des revendications 8 à 10, **caractérisé en ce que** ladite plaque comprend au moins deux têtons, chaque têton étant destiné à être engagé dans un évidement.

12. Ensemble selon l'une quelconque des revendications 8 à 11, **caractérisé en ce que** ladite plaque (34) comprend au moins deux ergots (35), de préférence diamétralement opposés, chaque ergot (35) étant destiné à être engagé dans un logement (33) de ladite pluralité de logements.

13. Ensemble selon l'une quelconque des revendications 10 à 12, **caractérisé en ce que** l'armature (12) comprenant un alésage central fileté (13), la partie filetée (39) de ladite vis (38) est en outre engagée dans ledit alésage central fileté (13), réalisant ainsi la solidarisation dudit instrument chirurgical (9) à ladite armature (12).

## Patentansprüche

1. Verstärkung (12) für die Basis einer Gelenkpfanne, umfassend ein geschwungenes Kreuz (1) mit mindestens vier konzentrischen Füssen (2), die um die Drehachse (3) verteilt sind, die der Achse der halben Sphäre des Gelenkpfannenhohlraums entspricht, und wobei unter den vier Füssen (2) mindestens ein erster Fuß (21) an seinem freien Ende (21 a) eine gekrümmte Spitze (17) umfasst, die ausgelegt ist, um im Lochverschluss des Beckens angebracht zu sein, wenn die Verstärkung (12) implantiert ist, mindestens einen zweiten Fuß (22), der ausgelegt ist, um auf der vorderen äußeren Seite des Gelenkpfannenhohlraums positioniert zu sein, wenn die Verstärkung (12) implantiert ist, umfassend an seinem freien Ende (22a) eine Platte (6), die im Wesentlichen oval und geschwungen ist, die ausgelegt ist, um auf dem Knochenträger fixiert zu sein, wobei der zweite Fuß (22) mit einem längsgerichteten Schlitz (7) ausgestattet ist, **dadurch gekennzeichnet, dass** der Schlitz auf der Ebene der ovalen und geschwungenen Platte (6) durch mindestens eine Öffnung (8) mit einer im Wesentlichen runden Form endet, und dass mindestens einer des dritten und vierten Fußes abreißbar ist.

2. Verstärkung (12) nach Anspruch 1, **dadurch gekennzeichnet, dass** der erste Fuß (21) und der zweite Fuß (22) einander gegenüber liegen und der dritte (23) und der vierte Fuß (24) abreißbar sind.

3. Verstärkung (12) nach Anspruch 1 bis 2, **dadurch gekennzeichnet, dass** jeder des oder der abreißbaren Fußes/Füße (23, 24) im Bereich seines freien Endes (23a, 24a) einen verengten Teil (23b, 24b) umfasst, der an seiner Stelle den Bruch des Fußes (23, 24) ermöglicht.

4. Verstärkung (12) nach einem der Ansprüche 1 bis 3, **dadurch gekennzeichnet, dass** der erste Fuß (21) im Bereich seines freien Endes (21 a) ein Loch (15) mit einer im Wesentlichen runden Form umfasst.

5. Einheit, umfassend eine Verstärkung (12) nach einem der Ansprüche 1 bis 4, wobei mindestens einer der Füße auf seiner inneren Fläche mit mindestens einer Aussparung (14) ausgestattet ist, wobei die Einheit außerdem mindestens einen Einsatz (5) umfasst, der ausgelegt ist, um sich aus einer verlängerten Position ohne Spannung in eine verengte Position ohne Spannung zu verformen, wobei der Einsatz (5) ausgelegt ist, um in die Aussparung (14) eingeführt zu werden, wenn er sich in seiner verengten Position befindet.

6. Einheit nach Anspruch 5, **dadurch gekennzeichnet, dass** der Einsatz (5) aus einem einzigen Teil in Drehform besteht, im Wesentlichen zylindrisch hohl, umfassend einen Schlitz (10) gemäß einem Generator parallel zu seiner Drehachse (11).

7. Einheit nach Anspruch 5 oder 6, **dadurch gekennzeichnet, dass** jeder Fuß (2, 21, 22, 23, 24) der Verstärkung (12) mit einer Aussparung (14) ausgestattet ist, in die ein Einsatz (5) eingeführt ist.

8. Einheit, umfassend eine Verstärkung (12) nach einem der Ansprüche 1 bis 4, wobei die Verstärkung auf ihrer inneren Seite mit mindestens einer Aussparung (16) ausgestattet ist, wobei die Einheit außerdem mindestens ein chirurgisches Instrument (9) zur Anbringung der Verstärkung (12) in einem Gelenkpfannenhohlraum umfasst, wobei das Instrument (9) mindestens einen Griff (25) und mindestens einen verlängerten Stift (26) umfasst, wobei der verlängerte Stift (26) an seinem distalen Ende mindestens einen Zapfen (36) zur ersten Positionierung des verlängerten Stifts (26) mit Bezug auf die Drehachse C, die der Achse der halben Sphäre der Gelenkpfannenhohlraums entspricht, umfasst, wenn der Zapfen (36) in die Aussparung (16) eingeführt ist, wobei das Instrument (9) außerdem Mittel zur Neupositionierung (27, 31, 33, 34, 35, 41) des verlängerten Stifts (26) mit Bezug auf die Drehachse C umfasst, wobei die Mittel zur Neupositionierung ausgelegt sind, um die Positionierung des verlängerten Stifts (26) mit Bezug auf die Drehachse C zu modifizieren, ohne den Zapfen (36) aus der Aussparung (16) zu entfernen.

9. Einheit nach Anspruch 8, **dadurch gekennzeichnet, dass** die Mittel zur Neupositionierung i) mindestens eine Platte (34) umfassen, die fest mit dem Zapfen (36) verbunden ist, wobei die Platte mit mindestens einem Sporn (35) ausgestattet ist, und ii) mindestens eine Schale (27), die fest mit dem distalen Ende des verlängerten Stifts (26) verbunden ist, wobei die Schale (27) an ihrem distalen Ende eine Sohle (31) umfasst, die auf ihrer distalen Seite (31a) mit einer Vielzahl von Aufnahmen (33) ausgestattet ist, die gemäß einem Kreis angeordnet sind, wobei die Platte (34) und die Schale (27) gemäß der Drehachse des Kreises ausgefluchtet und aneinander durch elastische Rückstellmittel (41) gekoppelt sind, wobei die elastischen Rückstellmittel ausgelegt sind, um sich aus einer Position unter erhöhter Spannung, in der die Platte (34) in Rotation mit Bezug auf die Schale (27) um die Drehachse des Kreises beweglich ist, in eine Position mit geringerer Spannung zu verformen, in der der Sporn (35) in mindestens eine Aufnahme (33) der Vielzahl von Aufnahmen eingeführt ist, wobei die Platte (34) dann in Rotation gegenüber der Schale (27) blockiert ist.

10. Einheit nach Anspruch 9, **dadurch gekennzeichnet, dass** die elastischen Rückstellmittel mindestens eine Spiralfeder (41) umfassen, die innerhalb einer zentralen zylindrischen Bohrung (29, 32) angeordnet ist, die die Schale (27) und die Sohle (31) durchquert, wobei die Platte (34) mit einer gewindegeschnittenen zentralen Öffnung (37) ausgestattet ist, die auf der zylindrischen Bohrung (29, 32) ausgefluchtet ist, wobei eine Schraube (38) auf der Spiralfeder (41) aufliegt, die innerhalb der zentralen zylindrischen Bohrung (29, 32) aufgenommen ist, wobei der gewindegeschnittene Teil (39) der Schraube (38) in die gewindegeschnittene zentrale Öffnung (37) der Platte (34) eingeführt ist, wobei die Spiralfeder (41) und die Schraube (38) somit die Kopplung mit Hilfe von elastischen Rückstellmitteln der Schale (27) an die Platte (34) durchführen.

11. Einheit nach einem der Ansprüche 8 bis 10, **dadurch gekennzeichnet, dass** die Platte mindestens zwei Zapfen umfasst, wobei jeder Zapfen ausgelegt ist, um in eine Aussparung eingeführt zu werden.

12. Einheit nach einem der Ansprüche 8 bis 11, **dadurch gekennzeichnet, dass** die Platte (34) mindestens zwei Sporne (35) umfasst, vorzugsweise einander diametral entgegengesetzt, wobei jeder Sporn (35) ausgelegt ist, um in eine Aufnahme (33) der Vielzahl von Aufnahmen eingeführt zu werden.

13. Einheit nach einem der Ansprüche 10 bis 12, **dadurch gekennzeichnet, dass** die Verstärkung (12) eine gewindegeschnittene zentrale Bohrung (13) umfasst, wobei der gewindegeschnittene Teil (39) der Schraube (38) außerdem in die gewindegeschnittene zentrale Bohrung (13) eingeführt ist, wodurch die feste Verbindung des chirurgischen Instruments (9) mit der Verstärkung (12) durchgeführt wird.

## Claims

1. A frame (12) for the base of an acetabulum comprising a curved cross (1) including at least four concentric legs (2) distributed around the axis of revolution (3) corresponding to the axis of the half-sphere of the acetabular cavity and, among said four legs (2), at least one first leg (21) including, at its free end (21 a), a bent tip (17) intended to be housed in the shutter hole of the pelvis when the frame (12) is implanted, at least one second leg (22), intended to be positioned in the anterolateral face of the acetabular cavity when the frame (12) is implanted, including at its free end (22a) a substantially oval and curved plate (6), intended to be fastened to the bony support, said second leg (22) being provided with a longitudinal slot (7), **characterized in that** said slot is terminated, at the level of said oval and curved plate (6), by at least one substantially circular-shaped orifice (8), and **in that** at least one of the third and fourth legs is self-breakable.

2. The frame (12) according to claim 1, **characterized in that** said first leg (21) and said second leg (22) are opposite to each other and said third (23) and fourth (24) legs are self-breakable.

3. The frame (12) according to any of claims 1 to 2, **characterized in that** each of said self-breakable leg(s) (23, 24) includes, in the region of its free end (23a, 24a), a narrowed portion (23b, 24b) enabling breakdown of said leg (23, 24) at its location.

4. The frame (12) according to any of claims 1 to 3, **characterized in that** said first leg (21) includes, in the region of its free end (21 a), a substantially circular-shaped hole (15).

5. An assembly comprising a frame (12) according to any one of claims 1 to 4, wherein at least one of said legs is provided with at least one recess (14) on its inner face, said assembly further comprising at least one insert (5) capable of being deformed from a de-constrained enlarged position to a constrained narrowed position, said insert (5) being intended to be engaged in said recess (14) when it is in its narrowed position.

6. The assembly according to claim 5, **characterized in that** said insert (5) consists of one single substantially cylindrical and hollow revolving part including a slot (10) along a generating line parallel to its axis of revolution (11).

7. The assembly according to claim 5 or 6, **characterized in that** each leg (2, 21, 22, 23, 24) of the frame (12) is provided with one said recess (14) wherein one said insert (5) is engaged.

8. An assembly comprising a frame (12) according to any of claims 1 to 4, said frame being provided, on its inner face, with at least one recess (16), said assembly further comprising at least one surgical instrument (9) for placing said frame (12) within an acetabular cavity, said instrument (9) comprising at least one handle (25) and at least one elongated rod (26), the elongated rod (26) comprising, at its distal end, at least one stud (36) for a first positioning of said elongated rod (26) with respect to the axis of revolution C corresponding to the axis of the half-sphere of the acetabular cavity, when said stud (36) is engaged in said recess (16), said instrument (9) further comprising means (27, 31, 33, 34, 35, 41) for repositioning said elongated rod (26) with respect to said axis of revolution C, said repositioning means being capable of modifying the positioning of said elongated rod (26) with respect to said axis of revolution C without disengaging said stud (36) from said recess (16).

9. The assembly according to claim 8, **characterized in that** said repositioning means comprise i) at least one plate (34) secured to said stud (36), said plate being provided with at least one spur (35), and ii) at least one shell (27) secured to the distal end of said elongated rod (26), said shell (27) comprising, at its distal end, a sole (31) provided on its distal face (31a) with a plurality of housings (33) disposed along a circle, said plate (34) and said shell (27) being aligned along the axis of revolution of said circle and being coupled to each other by elastic return means (41), said elastic return means being capable of being deformed from a highly constrained position, wherein said plate (34) is rotatably movable relative to said shell (27) about the axis of revolution of said circle, to a less constrained position, wherein said spur (35) is engaged in at least one housing (33) of said plurality of housings, said plate (34) being then rotatably blocked vis-a-vis said shell (27).

10. The assembly according to claim 9, **characterized in that** said elastic return means comprise at least one helical spring (41) housed within a central cylindrical bore (29, 32) passing through said shell (27) and said sole (31), said plate (34) being provided with a threaded central orifice (37) aligned on said cylindrical bore (29, 32), a screw (38) bearing on said helical spring (41) being received within said central cylindrical bore (29, 32), the threaded portion (39) of the screw (38) being engaged in the threaded central orifice (37) of said plate (34), said helical spring (41) and said screw (38) thereby coupling said shell (27) to said plate (34) by elastic return means.

11. The assembly according to any one of claims 8 to 10, **characterized in that** said plate comprises at least two studs, each stud being intended to be engaged in a recess.

12. The assembly according to any one of claims 8 to 11, **characterized in that** said plate (34) comprises at least two preferably diametrically opposite spurs (35), each spur (35) being intended to be engaged in a housing (33) of said plurality of housings.

13. The assembly according to any one of claims 10 to 12, **characterized in that** the frame (12) comprises a threaded central bore (13), the threaded portion (39) of said screw (38) is further engaged in said threaded central bore (13), thereby securing said surgical instrument (9) to said frame (12).
